# EUROPEAN PATENT APPLICATION

(11) **EP 4 445 838 A1**
(43) Date of publication of application: **16.10.2024**
(21) Application number: 22903646.2
(22) Date of filing: 09.12.2022
(51) Int. Cl.: A61B 5/11

(54) **DEVICE FOR DIAGNOSING NEUROLOGICAL PATHOLOGIES EXHIBITING TREMOR**

(30) Priority: 09.12.2021 ES 202131132
(71) Applicant: Román Martínez, Javier, 03400 Villena, Alicante (ES); Hernández Martínez, José Javier, 03540 Alicante (ES); Román Martínez, Ángel, 03400 Villena, Alicante (ES)
(72) Inventor: Román Martínez, Javier, 03400 Villena, Alicante (ES); Hernández Martínez, José Javier, 03540 Alicante (ES); Román Martínez, Ángel, 03400 Villena, Alicante (ES)
(74) Representative: Pons Ariño, Angel
(86) International application number: PCT/ES2022/070784
(87) International publication number: WO 2023/105107

(57) **Abstract**

The present invention allows a set of data concerning tremor frequency in a patient to be obtained, and guides the diagnosis of the neurological disease in a non-invasive manner. The invention comprises at least an enveloping cover (1), intended to envelope at least one hand of a patient, formed by at least a number of sleeves couplable to the fingers over the dorsal and distal area of the finger and a segment couplable to the palmar area of the wrist; first oscillation sensors (4) attached to the sleeves in correspondence with the dorsal and distal area of the finger, intended to measure an oscillation frequency, and second oscillation sensors (5) attached to the segment that is couplable to the palmar area, intended to measure an oscillation frequency. Furthermore, the invention discloses a method for measuring tremors, which uses the device.

## Description

### OBJECT OF THE INVENTION

The invention relates to a device that makes it possible to establish a presumptive diagnosis of the pathology underlying the sign exhibiting tremor, mainly presuming whether the tremor may be of physiological origin, an essential tremor or in the scope of rigid-akinetic syndromes (such as Parkinson's disease, progressive supranuclear palsy, multiple system atrophy, etc.). It guides the diagnosis of the neurological disease in a non-invasive manner, through a detailed analysis of the response of the different nerves that innervate the corresponding area of the hand and the palm. In this way, tests and resources can be adapted and guided towards the most appropriate diagnosis.

### BACKGROUND OF THE INVENTION

Tremors are oscillatory, involuntary and rhythmic movements of reciprocal, antagonistic muscle groups and they typically affect the hands, head, face, vocal cords, torso or legs.

Currently, the diagnosis of neurological pathologies exhibiting tremor is made by analysing family and personal medical history and the presence of other associated symptoms. It is often necessary to also perform blood tests and imaging tests to view the brain, such as a brain CT (computed tomography) or MRI (magnetic resonance imaging). Sometimes an electromyogram may also be requested.

Electromyography is a technique for graphically recording the electrical activity produced by skeletal muscles. It can be monitored through electrodes inserted in the muscles or through electrodes on the surface of the skin, over the muscle.

The diagnosis of tremor using electromyography is established by the characteristics of rhythmic muscle activity discharge (tremor), within range values that create a suspicion of the pathology.

Document CN111513724A is known from the state of the art and discloses a portable device suitable for monitoring and assessing the rehabilitation of the hand of a patient with Parkinson's disease. The device comprises a glove body, a signal acquisition unit arranged on the glove body, and an upper computer. The signal acquisition unit comprises a tremor signal acquisition unit, used to acquire tremor signals in the hands, a flexion signal acquisition unit used to acquire the degree of flexion of the hand, and a pressure signal acquisition unit used to acquire hand pressure.

The signal processing unit processes the signals acquired by the signal acquisition unit and transmits the signals to the upper computer, which determines the patient's degree of tremor through the tremor signals, rates the degree of flexion of the hand through a degree of flexion digital signal, and rates the degree of damage given the force of the hand muscles through a pressure digital signal.

As indicated in the description of the patent document, a flexion signal is used to measure tremor and another signal is used to measure hand pressure.

For its part, document CN104127187A discloses a system and method for the quantitative detection of cardinal signs of patients with Parkinson's disease. The system comprises a glove and a computer. The glove further comprises a wrist module and a fingertip module. The system further comprises a tremor detection module, a tremor amplitude steady-state detection module, a muscle stiffness detection module, and a slow movement symptom detection module.

The system presents a sum of three cardinal signs of Parkinson's disease and undergoes joint processing of the signals, which is analysed quantitatively by the computer.

Furthermore, document CN109620250A discloses a bracelet for detecting a risk of Parkinson's disease based on tremor. The bracelet includes a body, a detection system, a detection end, and a hand strap. The detection system includes a control unit, detection units, an information transmission unit, a storage unit, application software and a database. The bracelet can acquire a subject's physical data in real time and promptly determine whether there is a risk of Parkinson's disease; it also immediately reminds the subject to seek medical treatment.

Therefore, the device uses a bracelet for tremor analysis and it has a single sensor for subsequent processing.

Finally, document RU2483676C1 discloses a device for the complex analysis of different types of human tremors. The device comprises an accelerometer sensor, connected to an analogue-digital converter, and a computer with software for the input and processing of tremor signals. The device comprises a second monoaxial piezoelectric accelerometer, independent of the first. The device determines quantitative tremor parameters and compares them with the respective standard values.

In short, it is an individual sensor that analyses overall tremor without specifying the area of application on the body or on which structure the different accelerometer sensors are housed.

### DESCRIPTION OF THE INVENTION

The device for diagnosing neurological pathologies exhibiting tremor of the present invention obtains a set of data that allows a doctor or healthcare professional to establish a presumptive diagnosis of the pathology underlying the sign exhibiting tremor, mainly presuming whether the tremor is physiological, an essential tremor or in the scope of rigid-akinetic syndromes (such as Parkinson's disease, progressive supranuclear palsy, multiple system atrophy, etc.). Thus, the device guides the diagnosis of the neurological disease in a non-invasive manner, through a detailed analysis of the response of the different nerves that innervate the corresponding area of the hand and the palm. In this way, tests and resources can be adapted, providing an objective tool to the specialist in the diagnosis of the patient with tremor.

In addition, it optimises the subject's treatment since it establishes an initial value of the patient's tremor before starting the indicated therapy in each case, memorises the patient's data that is subsequently contrasted with reference values, and, in this way, the healthcare professional can see the previous and current situation before starting the medical/neurological consultation. The device is an objective tool for medical professionals that improves the diagnosis and management of patients with tremor, whether due to a degenerative origin, radiculopathy or a polyneuropathy underlying the tremor.

It is important to know that in the hands the median nerve innervates the thumb, index and middle fingers, and the ulnar nerve innervates the ring and little fingers. Furthermore, when the superior or inferior olivary complex is affected, an exacerbated tremor occurs that mainly affects the Y axis, and when the basal ganglia are affected, it relatively affects the X axis. Thus, by knowing how the tremor is exhibited, a specialist will be able to diagnose a patient suffering from this type of condition.

Specifically, the device comprises at least one enveloping cover, preferably two, right and left, intended to partially or completely envelope at least one hand of a patient. The enveloping covers are formed by a number of sleeves couplable to the fingers over at least the dorsal or distal area of the finger, and a segment couplable to the palmar area of the wrist.

Oscillation sensors are attached to the sleeves in correspondence with the dorsal and distal area and to the segment that is couplable to the palmar area, said sensors measuring the oscillation movement frequency in different axes, using hertz (Hz) as a unit of measurement and applying it to the horizontal (X) and vertical (Y) axes. In particular, the oscillation sensors are arranged at the distal end of the fingers on both hands, and in the palmar region of both wrists.

In a first aspect of the invention, the oscillation sensors comprise a high-precision gyroscope and an accelerometer, and the enveloping covers are gloves that facilitate the positioning of the sensors in contact with the patient. As indicated, these oscillation sensors detect the oscillation movement frequency of the five fingers and the palmar area, which helps a specialist diagnose the neurological disease.

In a second aspect of the invention, the oscillation sensors may comprise a set of additional elements, which improve the quality of the measurement, as well as detect a greater number of pathologies.

Specifically, the oscillation sensors additionally comprise an inclinometer, which, together with the accelerometers and the gyroscopes incorporated by each one, detect and stabilise the position, ruling out measurements of tremors that may be caused by causes unrelated to the pathology, such as when a person is nervous about taking the test.

Additionally, in this second aspect of the invention, the oscillation sensors may comprise a magnetic pulse generator and a magnetometer, which guides the device, as well as detects a magnetic field. Additionally, the oscillation sensors linked to the thumb and index fingers comprise a magnetic encoder, which converts the angular position of an axis into a digital code. In this way, once the measurements of tremors caused by causes unrelated to the disease have been filtered, the magnetometers and magnetic encoders measure the frequency with which the magnetic field is cut.

In this way, the frequency with which the fingers and the back of the hand cut the magnetic field is obtained, this being a pure measurement of movement of said finger or back of the hand, which is not affected by involuntary movements of the subject.

The objective of placing the oscillation sensors on the fingers is to assess the state of the nerves associated with each one. The median nerve innervates three of the fingers (thumb, index and middle fingers) and the ulnar nerve innervates the other two fingers (ring and little fingers). Furthermore, having a sensor on each finger and one on the wrist obtains more precise results for the assessment of the nerve underlying the pathology exhibited.

Furthermore, although the oscillation sensors could be placed on different parts of the body, the best way to measure oscillation is on the part furthest from the axis of motion. That is why the distal end of the hand is chosen. Furthermore, they are placed on the back of the fingers since the measurements are not affected by gravity and the materials of the enveloping covers; when the back of the fingers fall supported on a surface, it only relates to that surface. If the sensors were on the fingertips, they would experience a slight movement due to gravity during the study on position and, furthermore, if the enveloping covers were, for example, gloves, they would be affected by the distension of the glove's rubber.

In short, the twelve oscillation sensors are configured independently to obtain a frequency value in Hz, for the X-axis and for the Y-axis preferably. In addition, the sensors can be linked to a processor, configured to calculate the mean of each sensor independently and of both hands to obtain reference values. The device, connected to the processor, may comprise a power module and a module for connecting and sending data to an external device.

Additionally, the device may comprise other types of sensors, such as pressure, motion change, temperature or sweat sensors, attached to the enveloping covers. In this way, the device collects a larger set of data of interest, which can help the specialist make the diagnosis.

Furthermore, another object of the invention is a method for measuring tremors of a patient, which uses the device for diagnosing neurological pathologies exhibiting tremor, described above. The method also obtains a set of patient data, which is compared with threshold values, and which a specialist may be able to interpret in order to diagnose the disease affecting the patient.

Specifically, the frequency measurements determined by each of the device's twelve sensors are first obtained in the method. Preferably, they are obtained in the X and Y axes. Next, the mean and optionally the variance of the frequency measurements are obtained. Afterwards, the different values obtained from the sensors are compared to contrast them with the possible diseases known to date. The lower the variance of the data, the greater the possibility of getting closer to the diagnosis than if the dispersion is more significant.

This comparison is shown to the specialist, and based on this comparison, they are able to determine the disease affecting the patient.

The threshold values used for the comparison can be obtained from a database, which stores the results obtained after subjecting other patients to this test.

Furthermore, the method can provide an additional parameter. In the case of diseases exhibiting tremor, the diagnosis may depend on the age range in which the patient finds themselves. In other words, if a patient is above an age range, it is considered that they will likely suffer from the disease associated with the range of threshold values, and if they are below that age range, it is only possible that the patient will suffer from it.

For example, in the case of a patient over the age of sixty years, the fact that they will suffer from a disease associated with tremors is likely, while if they are under the age of sixty years, it is possible. Therefore, in one stage of the method, information on the patient's age can be obtained, and depending on whether the age is above or below a certain pre-established range, it can be indicated whether it is probable or possible that the patient suffers from that disease.

In this way, the method calculates variations with respect to the standard to help the specialist make a presumptive diagnosis that the specialist must further analyse.

### DESCRIPTION OF THE FIGURES

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:
Figure 1 shows the dorsal area of the glove with the Velcro on the distal position of the fingers.
Figure 2 shows the palmar area of the glove with the Velcro on the palmar area.
Figure 3 shows the dorsal area of the glove with the sensors and the processor attached to the Velcro.

### PREFERRED EMBODIMENT OF THE INVENTION

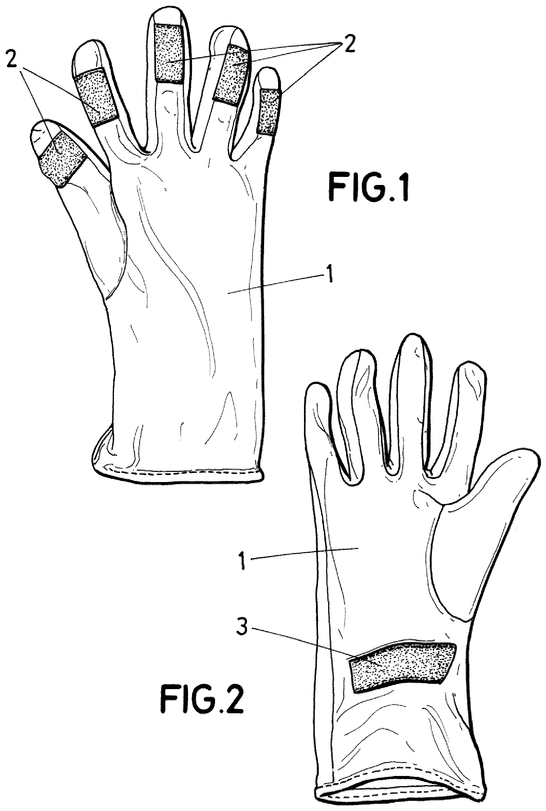

A preferred embodiment of the device for diagnosing neurological pathologies exhibiting tremor, and of a method for measuring tremors, which uses the device, is described below with the help of figures 1 to 3.

As shown in figures 1 and 2, the device comprises right and left enveloping covers, particularly gloves (1), one intended for the right hand and another for the left hand of a patient. As shown in figure 1, in the distal area of the fingers, the gloves (1) comprise first attachment elements (2), preferably Velcro, arranged in the area of the back of the hand. Furthermore, as shown in figure 2, the gloves (1) comprise second attachment elements (1), arranged on the palmar side of the hand, preferably Velcro.

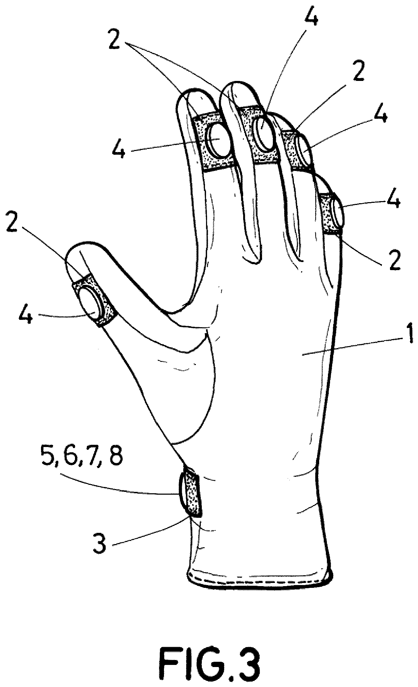

As shown in figure 3, finger oscillation sensors (4) and wrist oscillation sensors (5) are affixed to the attachment elements (2, 3), said sensors comprising a high-precision three-axis gyroscope and a three-axis accelerometer. The oscillation sensors (4, 5) additionally comprise a magnetometer, an inclinometer and a pulse generator. Furthermore, the oscillation sensors (4, 5) intended to be placed on the thumb and index fingers of the hand comprise a magnetic encoder.

Accelerometers, gyroscopes and inclinometers detect, stabilise and emit the position of the hand without being affected by sudden movements during the measurement. Furthermore, once the effect of hand movement is cancelled out, the magnetometers and the magnetic encoders measure the cut-off frequency generated by the different fingers, as well as the cut-off frequency generated by the back of the hand.

In this way, the oscillation sensors (4, 5) measure the frequency in Hertz with which the fingers and the back of the hand cut the magnetic field, this being a pure measure of movement of said finger or back of the hand, without being affected by involuntary movements of the subject.

Furthermore, the device comprises a processor (7), arranged in the wrist attachment element (3), which provides the mean value of the frequencies obtained in the right hand and the left hand. The device, linked to the processor (7), comprises a power module (8) and a module for connecting and sending data (6) to an external device, which can send data, for example, via USB, Bluetooth, Wi-Fi and 4G/5G, among others.

The gloves (1) may further comprise an extendible rubber band to adjust to the size of the hand.

Furthermore, it is important to explain the reason for the preferred use of all the defined sensors (accelerometer, gyroscope, magnetometer, inclinometer, pulse generator and encoder) for measuring any type of tremor. To this end, the medical concept of tremor is defined below.

Tremor is defined as an involuntary, rhythmic and oscillatory movement produced by repetitive, synchronous or alternating contractions of antagonist muscles. To establish a syndromic/etiological diagnosis of tremor, the following approaches should be taken to determine whether the movements:
1) appear when the subject is at rest or performing actions (when maintaining postures or making movements);
2) have a sudden and self-limited onset or are persistent;
3) have a circadian occurrence pattern (e.g., appear in the afternoon, persist during sleep);
4) there are or are not precipitating factors (e.g., standing up, writing);
5) can be voluntarily controlled for a period of time.

During the neurological examination, a detailed observation of the movements will be carried out in order to establish whether the tremor criteria are met and to know their location (proximal, distal, unilateral, segmental), rhythm (and frequency), amplitude, speed and pattern. The degree of control by will and the possible aggravating/precipitating factors described by the patient will also be confirmed.

Once the rhythmicity of the movement has been identified, during the examination it should be checked whether it appears when the subject is at rest or performing actions (postural and/or kinetic). To do this, it must first be verified that the patient's limbs are completely relaxed and at rest.

The examination of action tremor is based on manoeuvres that cause tremor. To examine postural tremor, the patient is asked to extend their upper limbs and hold them in front of them. They are asked to do the same with their lower limbs. Kinetic tremor is examined with the finger-nose test, in which it is observed whether the tremor is maintained throughout the entire movement or increases as it reaches the target.

To further examine the patient, writing and spiral drawing tests that will provide more information are carried out. Orthostatism should also be examined to check that there is no tremor while the patient is standing. If the patient complains of tremor during a specific task, this task should be performed during the consultation (for example, drinking from a glass, writing, or using eating utensils) as it can guide the etiological diagnosis.

The sensors described above are those that obtain the qualities expressed above. In other words, they determine the location (proximal, distal, unilateral, segmental), rhythm (and frequency), amplitude, speed and pattern of the tremor.

Furthermore, another object of the invention is a method for measuring tremors, which uses the device described above, and which obtains the oscillation frequencies of a patient's hands, which are compared with threshold values and which help a specialist diagnose the neurological disease exhibiting tremor.

Specifically, the values of the frequencies measured by each of the twelve sensors (4, 5) in the X and Y axes are first obtained in the method, thanks to the accelerometers, gyroscopes and inclinometers which detect and stabilise the position of the fingers and palm, ruling out tremors unrelated to the subject's pathology. The frequency with which the fingers and the back of the hand cut the magnetic field is then measured with the magnetometers and the magnetic encoders. Next, the mean of the frequency values is obtained. Afterwards, the calculated mean is compared with frequency threshold values.

Based on the comparison with the reference threshold values, the measurements obtained are classified in a pre-established range of values. This range can be useful to a specialist when making their diagnosis.

The threshold values used to establish the presumptive diagnosis have been previously obtained after analysing a large number of patients with the different pathologies on which the device works, establishing a database against which the results of the measurements can be compared.

Moreover, in an additional step, it can be indicated whether the measurements taken with the device will be postural measurements or measurements at rest. The measurement at rest consists of the patient resting their hands on a horizontal surface until all the fingers are measured in this state. The postural measurement consists of the patient extending their arms to a height of 20 centimetres, without a horizontal surface to rest on, until all the fingers are measured in this state.

This differentiation and act of taking measurements in two different positions is important, since there are tremors that occur at rest and not in position, and vice versa; the sum of the information in both cases helps to classify the case.

Therefore, the method of the invention and the ranges in which it classifies tremors allow a specialist to determine whether the patient suffers from: Essential or physiological tremor, Parkinson's disease, Holmes tremor or Mesencephalic or Thalamic tremor, or Peripheral neuropathy that can be median or ulnar neuropathy.

## Claims

1. A device for diagnosing neurological pathologies exhibiting tremor, comprising:
- at least one enveloping cover (1), intended to partially or completely envelope at least one hand of a patient, which is formed by at least:
- a plurality of sleeves couplable to the fingers over at least the dorsal and distal area of the finger, and
- a segment couplable to the palmar area of the wrist,
- five first oscillation sensors (4) attached to each of the sleeves in correspondence with the dorsal and distal area of the finger, intended to measure an oscillation frequency, and
- a second oscillation sensor (5) attached to the segment that is couplable to the palmar area, intended to measure an oscillation frequency,
each of the oscillation sensors (4, 5) comprising a gyroscope and an accelerometer.

2. The device of claim 1, wherein the at least one enveloping cover (1) is a glove.

3. The device of claim 2, wherein the gloves additionally comprise size adjustment elements.

4. The device of claim 1, wherein the oscillation sensors (4, 5) are attached to the at least one enveloping cover (1) with Velcro (2, 3) arranged on the sleeves couplable to the fingers and to the couplable segment.

5. The device of claim 1, wherein the oscillation sensors (4, 5) additionally comprise a magnetometer, an inclinometer and a pulse generator.

6. The device of claim 5, wherein the oscillation sensors (4, 5) attached to the sleeves in correspondence with the dorsal and distal area of the thumb and index fingers additionally comprise a magnetic encoder.

7. The device of claim 1, comprising two enveloping covers (1).

8. The device of claim 1, additionally comprising a processor (7) connected to the oscillation sensors (4, 5) and configured to calculate the mean of the measured oscillation frequencies.

9. The device of claim 1, additionally comprising a module for connecting and sending data (6), said module linked to the oscillation sensors (4, 5).

10. The device of claim 1, additionally comprising a power module (8) connected to the oscillation sensors (4, 5).

11. The device of claim 1, additionally comprising one or more sensors selected from: pressure sensor, motion change sensor, temperature sensor and sweat sensor.

12. A method for measuring tremors, which uses the device of any of the preceding claims, and which comprises the steps of:
- receiving, from the oscillation sensors (4, 5), oscillation frequency measurements in the fingers and wrists of a patient,
- calculating the mean of the oscillation frequency measurements, and
- comparing the calculated mean with pre-established ranges of oscillation frequencies.

13. The method of claim 12, wherein the oscillation frequency measurements are obtained on an X axis and a Y axis.
